# FASCICULE DE BREVET EUROPEEN

(11) **EP 2 911 811 B1**
(45) Date de publication et mention de la délivrance du brevet: **01.02.2017**
(21) Numéro de dépôt: 13820896.2
(22) Date de dépôt: 24.10.2013
(51) Int. Cl.: B09B 1/00, B03B 9/06, B09B 5/00

(54) **LIGNE DE TRAITEMENT DE DÉCHETS CONSTITUÉS D'ORDURES MÉNAGÈRES ET/OU DE BIODÉCHETS**
ANLAGE ZUR BEHANDLUNG VON AUS HAUSMÜLL UND/ODER BIOMÜLL BESTEHENDEM ABFALL
LINE FOR TREATING WASTE CONSISTING OF HOUSEHOLD WASTE AND/OR BIOWASTE

(30) Priorité: 26.10.2012 FR 1260211
(43) Date de publication de la demande: 02.09.2015
(73) Titulaire: SUEZ Groupe, 92040 Paris la Défense Cedex (FR)
(72) Inventeur: FLEURY, Sylvie, F-78125 Orphin (FR)
(74) Mandataire: Cabinet Armengaud Aîné
(86) Numéro de dépôt international: PCT/IB2013/059614
(87) Numéro de publication internationale: WO 2014/064639

(56) Documents cités:
- WO-A1-2006/059319
- GB-A- 435 380

## Description

L'invention est relative à une ligne de traitement de déchets constitués d'ordures ménagères résiduelles et/ou de biodéchets, ligne de traitement en vue de séparer la matière organique et les matières à haut PCI (Pouvoir Calorifique Inférieur), du genre de celles qui comprennent :
- des moyens de stockage des déchets,
- des moyens de séparation de matières valorisables,
- des moyens de tri entre des produits de granulométries différentes,
- des moyens pour diriger les produits de plus forte granulométrie vers au moins une unité de traitement, notamment de valorisation énergétique,
- et au moins un digesteur pour la méthanisation de produits à plus forte teneur organique, de granulométrie plus faible.

Les déchets concernés par la ligne de traitement comprennent notamment les déchets ménagers, la fraction fermentescible des déchets, les biodéchets provenant de collectes sélectives, la pulpe de biodéconditionneurs de déchets industriels, et les mélanges de déchets organiques urbains, agricoles et industriels.

Le document WO 2006/059319 A1 décrit un digesteur comprenant un tambour rotatif dans lequel sont traités des déchets.

Une ligne de traitement traditionnelle inclut une étape de préparation des déchets (tri, broyage,...), une étape de méthanisation par voie sèche ou humide, et une étape de traitement des digestats provenant de la méthanisation des déchets, par voie traditionnelle de compostage et/ou de stabilisation et/ou d'épandage et/ou de bioséchage en tunnel, en andain ou sous sac.

La méthanisation est un procédé anaérobie de dégradation de la matière organique dite putrescible contenue dans les déchets ménagers, qu'ils soient bruts ou biodéchets issus de collectives sélectives. Un pré-tri est effectué en amont du digesteur pour la méthanisation, et est adapté en fonction du procédé de méthanisation envisagé. Différentes fractions granulométriques sont ensuite destinées à des procédés de traitement organique ou thermique, type stabilisation, compostage ou bioséchage.

Les déchets ménagers et biodéchets sont de granulométries et de qualités très hétérogènes. L'hétérogénéité de la matière, même après un tri, est complexe et instable. Les matières constituant le digestat sortant du digesteur, contiennent des inertes tels que du verre, des pierres, ainsi que des matières plastiques et éventuellement des traces de métaux ferreux et non ferreux. La majeure partie est cependant organique et énergétique et le traitement de ce digestat est généralement assuré par compostage, stabilisation et/ou bioséchage.

Le traitement de bioséchage, par exemple de type « Tunnel » ou « andains », requiert des surfaces au sol importantes. Les flux hydriques et odorants sont difficiles à gérer. Ce traitement s'accompagne de fortes émanations d'odeurs et nécessite une gestion complexe des jus non canalisés.

La digestion par voie sèche gère des déchets hétérogènes dont la siccité est comprise entre 30 et 50 %, avantageusement 37 %. La siccité en sortie de digestion est de l'ordre de 22 à 40 %, avantageusement de 28 %. Les digestats produits sont parfois déshydratés, par pressage ou autre technologie, notamment centrifugation, et sont ensuite destinés au compostage, ou encore épandus, stabilisés ou bioséchés.

La digestion par voie humide, qui fait parfois intervenir une hydrolyse préalable, présente les mêmes caractéristiques de siccité de digestats sortants comprise entre 12 et 5 %, avantageusement égale à 6 ou 7 %. Une production de jus excédentaires sur site est souvent source d'odeurs et de traitements spécifiques complémentaires. Les mêmes contraintes s'appliquent sur les filières aval.

De telles solutions présentent plusieurs inconvénients.

Une surface foncière importante est requise, en particulier pour le bioséchage en tunnel. En coeur de ville, il est souvent difficile d'acquérir les surfaces nécessaires pour un tel traitement de bioséchage.

Le problème des odeurs sur le site et à la périphérie du site est difficile à résoudre avec les traitements traditionnels extensifs des digestats. Ces procédés ne permettent pas de répondre aux exigences, concernant les odeurs, sans des mises en oeuvre contraignantes de génie civil avec bâtiments.

Les conditions d'exploitation, en termes de polluants gazeux d'aérosols et de micro-organismes aériens, sont difficiles à l'égard de la santé des opérateurs. Les exploitants d'unités de méthanisation/compostage des déchets sont soumis à des contraintes d'exploitation fortes, telles que le port d'un masque obligatoire. Des flux chargés en polluants, même s'ils sont traités et renouvelés, sont parfois difficiles à supporter. L'imprégnation des odeurs dans les halls et tunnels de traitement des digestats est forte. L'exposition à l'ammoniac est contrôlée mais toujours difficile à accepter.

Les débits d'air nécessités par une technologie de bioséchage en tunnel sont importants et consommateurs d'énergie.

L'invention a pour but, surtout, de remédier au moins en partie aux inconvénients évoqués ci-dessus, et notamment de minimiser la surface requise pour le traitement des déchets et d'optimiser les conditions environnementales, en particulier concernant les odeurs, et sanitaires concernant les exploitants.

Selon l'invention, une ligne de traitement de déchets du genre défini précédemment est caractérisée en ce que :
- les moyens de tri sont prévus pour fournir une fraction de produits de granulométrie inférieure à 20 mm,
- au moins un digesteur est affecté à la méthanisation de cette fraction de granulométrie inférieure à 20 mm,
- et la ligne de traitement comprend au moins un sécheur thermique basse température, généralement inférieure à 95°C, qui est alimenté par le digestat issu de la fraction de granulométrie inférieure à 20 mm.

Avantageusement, les moyens de tri sont prévus pour fournir une fraction de produits de granulométrie 0-10 mm, au moins un digesteur est affecté à la méthanisation de cette fraction de granulométrie 0-10 mm, et le sécheur thermique basse température est alimenté par le digestat de ce digesteur.

De préférence, la ligne de traitement comporte :
▪ une première branche pour les matières de granulométrie supérieure, comprenant :
   - au moins un premier digesteur (A) affecté à la méthanisation desdites matières de granulométrie supérieure,
   - au moins une centrifugeuse ou une presse à laquelle est soumis le digestat du premier digesteur,
   - au moins un tunnel de bioséchage pour les matières issues de la centrifugeuse ou de la presse,
▪ une deuxième branche pour les matières de granulométrie inférieure, comprenant :
   - au moins un deuxième digesteur (B) affecté à la méthanisation des matières de granulométrie inférieure,
   - au moins un mélangeur entre le digestat du deuxième digesteur (B) et les boues de la centrifugeuse, ou le pressat, de la première branche,
   - et au moins un sécheur thermique intensif auquel est soumis le mélange issu du mélangeur.

La ligne de traitement comporte avantageusement :
- des moyens de tri assurant une production de fractions 0-60 mm, 10-20 mm, et 0-10 mm.
- au moins un premier digesteur (A) pour les fractions 0-60 mm et 10-20 mm qui sont méthanisées avec du structurant, notamment fibres végétales, puis soumises à une presse et/ou une centrifugeuse pour la production de gâteaux de presse et/ou de boues de centrifugation,
- au moins un deuxième digesteur (B) pour la fraction 0-10 mm qui est méthanisée sans structurant et n'est pas déshydratée en sortie,
- un mélangeur pour mélanger le digestat issu du deuxième digesteur avec les boues de centrifugation du digestat provenant du premier digesteur (A)
- au moins un sécheur thermique intensif basse température dans lequel est séché le mélange sortant du mélangeur ou du digesteur,
- et au moins un tunnel de maturation/bioséchage pour le gâteau de presse de la fraction 0-60 mm et 10-20 mm.

La ligne de traitement peut comporter :
- un premier tambour séparateur dont les mailles correspondent à une granulométrie comprise entre 100 et 500 mm, notamment égale à 300 mm
- un poste de valorisation énergétique du refus du premier tambour séparateur,
- un deuxième tambour séparateur à mailles plus petites que celles du premier tambour, notamment à mailles de 60 mm, auquel sont envoyés les passants du premier tambour,
- un fermenteur auquel est envoyé le refus du deuxième tambour, après être passé par une étape de tri qui extrait les produits métalliques, notamment les produits ferreux,
- un troisième tambour séparateur, dont les mailles correspondent à une granulométrie de 20 mm, qui reçoit les produits sortant du fermenteur, le refus du troisième tambour étant dirigé vers le poste de valorisation énergétique, - des moyens pour séparer les passants, ayant traversé les mailles du troisième tambour, en une fraction de granulométrie comprise entre 10 et 20 mm, dirigée vers un mélangeur et une fraction de granulométrie 0-10 mm, dirigée vers le deuxième digesteur.

Le mélangeur, qui reçoit la fraction de granulométrie comprise entre 10 et 20 mm, reçoit également les passants qui ont traversé le deuxième tambour séparateur, les produits mélangés sortant du mélangeur étant introduits dans le premier digesteur.

Dans le cas d'une ligne de traitement pour biodéchets, cette ligne comporte avantageusement :
- un broyeur de biodéchets,
- de moyens de criblage pour fournir une fraction de produits de granulométrie inférieure à 20 mm et une fraction de produits de granulométrie supérieure,
- au moins un digesteur affecté à la méthanisation des produits de granulométrie inférieure à 20 mm,
- et un sécheur thermique intensif auquel est soumis le digestat du digesteur, après traversée éventuelle d'une centrifugeuse ou d'une presse.

Un sécheur thermique basse température comporte des moyens de soufflage d'air chaud, à une température généralement inférieure à 95°C et supérieure à 60°C, sur les matières à sécher disposées sur au moins une bande convoyeuse, de préférence perméable à l'air, et un circuit fermé de distribution de l'air de séchage, l'ensemble étant confiné dans une enceinte fermée, comportant une entrée pour les produits à sécher, et une sortie pour les produits séchés.

Le sécheur thermique peut être piloté pour que la siccité finale en sortie du sécheur thermique soit comprise entre 50 et 90 %, en fonction des attentes de l'exploitation

La mise en place d'un tel sécheur thermique basse température permet de canaliser les flux, notamment le flux ammoniacal, dans un équipement industriel confiné et de moduler la siccité de sortie selon les usages.

L'emprise au sol d'un tel sécheur thermique intensif est très inférieure à celle d'un bioséchage en tunnel.

Malgré le caractère hétérogène des déchets traités par l'invention, et la présence d'indésirables et d'inertes, la préparation d'un digestat à partir d'une fraction de faible granulométrie déterminée a permis de traiter ce digestat par un sécheur thermique intensif essentiellement utilisé pour des produits homogènes, surtout quant à la granulométrie, et dépourvus d'inertes.

La ligne de traitement permet de traiter différents types de déchets, à savoir déchets urbains, municipaux, et/ou biodéchets, issus d'une collecte sélective organique, et/ou mélanges de substrats urbains, industriels, voire agricoles.

Selon l'invention, le tri des matières en amont permet de produire un digestat plus fin de rhéologie adapté au traitement thermique intensif.

Dans le cas où les déchets sont constitués d'ordures ménagères résiduelles, la ligne de traitement est prévue pour distinguer les destinations organiques des matières brutes entrantes, à savoir distinguer un compostage normé d'un simple stabilisat.

L'invention combine des refus de centrifugation de jus de digestion avec les digestats plus fins de fractions comprises entre 5 et 20 mm, selon les procédés de traitement. On peut donc, sur un même site, substituer une partie de bioséchage en tunnel par une partie de séchage intensif basse température, et réduire ainsi considérablement l'emprise foncière.

En bioséchage traditionnel, la siccité sortante des digestats est de 40 à 55 %. Il est extrêmement difficile et coûteux en termes de consommation d'énergie de vouloir dépasser cette siccité.

Selon l'invention, avec un sécheur thermique intensif, la siccité finale peut être pilotée en fonction des attentes de l'exploitation :
- si le site est en centre ville et que les digestats doivent être valorisés par voie agronomique, un compostage s'imposera hors du site, et les digestats seront séchés entre 50 et 90 %, avantageusement 65 %. Ces digestats seront compostés après transport.
- si le site est en centre ville et qu'il est souhaitable d'utiliser les digestats par voie énergétique ou chimique, une siccité optimale entre 70 et 90 % sera recherchée en sortie du sécheur intensif basse température.

Cette modularité de traitement, et la large gamme de siccité possible sont nouvelles et inattendues par rapport aux techniques classiques de traitement des déchets en question.

En outre, l'utilisation de digestats séchés thermiquement dans un site de compostage de déchets posait problème. En effet, il faut pouvoir juger de la bonne siccité qui maintient une flore biologique minimale, apte à se réactiver en compostage, ou encore une matière organique suffisamment accessible pour l'activité biologique du compostage. Selon l'invention, les conditions de compostabilité (ajout de structurants, type de structurants, quantités, paramètres biochimiques...), la compressibilité du mélange (détermination de l'espace lacunaire ou porosité), la durée de compostage (besoins en aération, cinétique...) et l'évolution de maturité au cours du processus sont évaluées au cours du séchage thermique intensif afin de bien définir la siccité optimale des matières en sortie du sécheur thermique.

L'invention consiste, mises à part les dispositions exposées ci-dessus, en un certain nombre d'autres dispositions dont il sera plus explicitement question ci-après à propos d'exemples de réalisation décrits avec référence aux dessins annexés, mais qui ne sont nullement limitatifs. Sur ces dessins :
Fig. 1 est un schéma bloc simplifié d'une ligne de traitement de déchets selon l'invention.
Fig.2 est un schéma plus détaillé d'une partie de la ligne de traitement de Fig.1.
Fig. 3 est un schéma bloc d'une autre partie de la ligne de traitement de déchets.
Fig. 4 est un schéma de la partie de la ligne de traitement comportant le sécheur thermique intensif, et
Fig. 5 est un schéma bloc d'une ligne de traitement de biodéchets selon l'invention.

Pour une meilleure compréhension de la description, un lexique relatif à des termes utilisés est donné ci-après.

Fermenteur = réacteur de brassage de la matière pendant un temps donné pour assurer une séparation matière et préparer une partie fermentescible à la digestion.

Digesteur = enceinte fermée dans laquelle des micro-organismes majoritairement bactériens assimilent la matière, en anaérobiose, et produisent du biogaz, et un résidu appelé digestat.

Digestat = matière sortant du digesteur, sous forme liquide ou pâteuse, pouvant comporter des matières solides en suspension, parmi lesquelles de la matière organique, des inertes et des indésirables.

Biogaz = produit gazeux émanant de réactions biochimiques de décomposition de la matière organique dans le digesteur.

CSR = combustible solide de récupération.

A titre de précision non limitative, les compositions des matières constituant les déchets sont les suivantes :

### OMR : ordures ménagères résiduelles

Il s'agit des déchets ménagers constitués de déchets collectés auprès des ménages, ainsi que des déchets assimilables aux déchets ménagers d'origine commerciale ou artisanale dans la mesure où ils sont collectés conjointement avec les déchets des ménages.
Matières sèches : 30 à 40 % en poids
Matières organiques non synthétiques/matières sèches : 40 %
Matières organiques/matières sèches : 55 à 70 %
   jusqu'à 40 % en poids de fermentescibles
   jusqu'à 17 % en poids de textiles
   jusqu'à 12 % en poids de matières plastiques
Verres : 3 à 11 % en poids
Métaux : 2 à 5 % en poids
Inertes : 5 % en poids

### Biodéchets issus de la collecte sélective des ménages

Matières sèches (siccité) 20 à 45 % en poids
Matières organiques/matières sèches : 60 à 90 %
Taux d'indésirables : de 2 à 10 % en poids
Indésirables : verres, métaux, matières plastiques, fibres textiles. Parmi les indésirables, les verres représentent environ 5 % en poids.

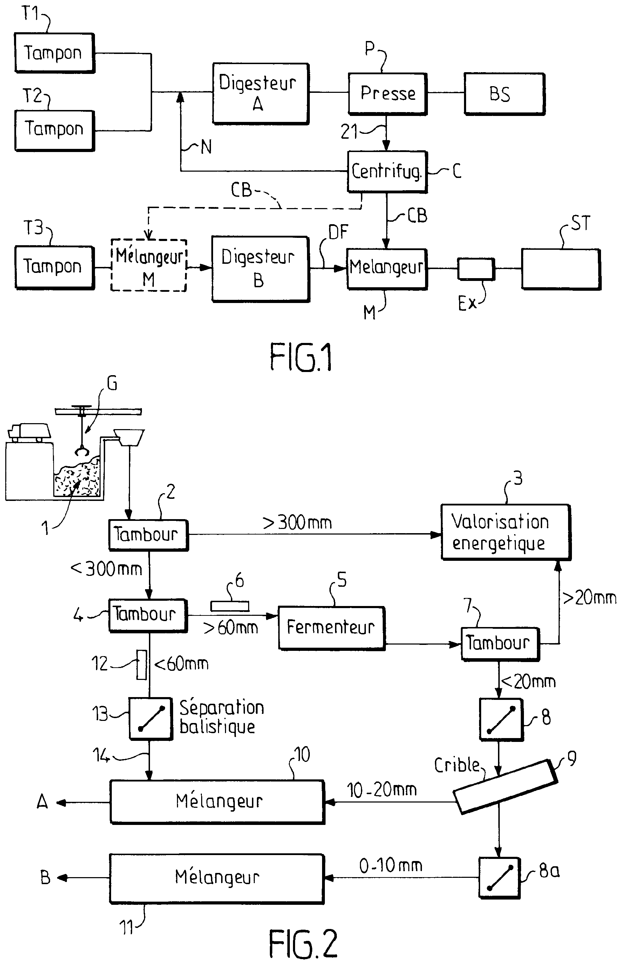

En se reportant à Fig. 1 des dessins, on peut voir un schéma bloc simplifié d'une ligne de traitement selon l'invention pour ordures ménagères résiduelles et éventuellement biodéchets. Les matières ont subi un tri en amont, et une fosse tampon T1 contient des matières qui ont une granulométrie comprise entre 0-60 mm et des matières qui, après être passées dans un fermenteur et avoir subi un deuxième tri, ont une granulométrie comprise entre 10-20 mm. Une fosse T2 est dédiée aux biodéchets ou aux déchets verts, après broyage et criblage éventuels.

Les matières prélevées dans les fossesT1,T2 sont introduites dans un digesteur A d'une première branche de la ligne de traitement. Le digesteur A produit du biogaz évacué par une sortie non représentée, et un digestat dirigé vers une presse P, notamment une presse à vis, dont le gâteau de pressage est envoyé dans un tunnel de bioséchage BS, tandis que le jus ou pressat est envoyé dans une centrifugeuse C. Le jus de la centrifugeuse est recirculé selon un trajet N à l'entrée du digesteur A. Les boues de centrifugation CB sont dirigées vers un mélangeur M.

Une fosse tampon T3 sert de stockage intermédiaire à une fraction de matières de granulométrie comprise entre 0-10 mm. Ces matières sont introduites dans un digesteur B d'une deuxième branche de la ligne de traitement. Le digesteur B donne en sortie un digestat fin DF qui est envoyé dans le mélangeur M pour former, avec les boues de centrifugation CB, un mélange relativement homogène. Ce mélange sort du mélangeur M et est dirigé vers un sécheur thermique intensif ST, après avoir éventuellement subi une extrusion dans un extrudeur EX propre à conditionner la matière sous forme de cordons de quelques centimètres de long favorisant le séchage.

En variante, comme illustré en tirets sur Fig.1, le mélangeur M peut être situé en amont du digesteur B ; dans ce cas, le digesteur B traite en mélange les matières de la fosse tampon T3, avec les boues de centrifugation CB, et le digestat fin DF est dirigé vers le sécheur thermique intensif ST, en passant éventuellement par l'extrudeur EX.

Le sécheur thermique ST est un sécheur à circulation d'air chaud en circuit fermé pour sécher les matières qui circulent sur au moins une bande convoyeuse, de préférence perméable à l'air. Le sécheur comporte une enceinte avec entrée pour les produits à sécher et sortie pour les produits séchés. Des moyens de chauffage de l'air de séchage sont prévus, ainsi que des moyens de récupération de chaleur, notamment par condensation de la vapeur d'eau. La température de l'air chaud de séchage est inférieure à 95°C, généralement de l'ordre de 80 à 85°C, et supérieure à 60°C.

Sur Fig. 2, on peut voir la filière de tri de la ligne de traitement de déchets. Une fosse 1 ou une dalle est prévue pour le stockage des déchets qui sont prélevés par un engin de préhension G pour être introduits dans un tambour séparateur 2 (ou Trommel) dont les mailles correspondent à une granulométrie importante, notamment égale à 300 mm ou plus. Les mailles du tambour peuvent être circulaires, polygonales ou oblongues. La valeur indiquée de 300 mm n'est pas limitative, et cette valeur peut être comprise entre 100 et 500 mm selon les cas.
Le refus du tambour 2, dont la granulométrie est supérieure aux dimensions des mailles, est dirigé vers un poste 3 de valorisation énergétique du refus CSR (combustible solide de récupération).

Les passants, c'est-à-dire les matières qui ont traversé les mailles du tambour 2, sont envoyés dans un deuxième tambour séparateur 4 à mailles plus petites que celles du tambour 2, notamment à mailles de 60 mm. Le refus du tambour 4, constitué par des produits de granulométrie supérieure à 60 mm selon l'exemple non limitatif donné, est dirigé vers un fermenteur 5, après être passé par un poste de tri 6 qui extrait les produits métalliques, notamment les produits ferreux.

Les produits sortant du fermenteur 5 sont introduits dans un troisième tambour séparateur 7 dont les mailles sont plus petites que celles du tambour 4 et correspondent à une granulométrie de 20 mm. Le refus du tambour 7, correspondant à des produits ayant une granulométrie supérieure à 20 mm, est dirigé vers l'unité 3 de valorisation énergétique. Les passants, ayant traversé les mailles du tambour 7, sont constitués principalement de matières organiques d'une granulométrie inférieure à 20 mm et sont dirigées vers un séparateur balistique 8 suivi d'un crible trampoline 9, à mailles de 10 mm. Les refus du crible 9, correspondant à une granulométrie comprise entre 10 et 20 mm, sont dirigés vers un mélangeur 10. Les passants du crible 9, correspondant à une granulométrie de 0-10 mm, sont dirigés vers un séparateur balistique 8a, puis vers un mélangeur 11.

Les passants du tambour séparateur 4, d'une granulométrie inférieure à 60 mm, sont dirigés vers le mélangeur 10, en traversant un poste de tri 12 de produits métalliques, notamment ferreux, puis un séparateur balistique 13 qui écarte les produits les plus denses, notamment les produits minéraux. Les passants 14 sont essentiellement organiques et introduits dans le mélangeur 10.

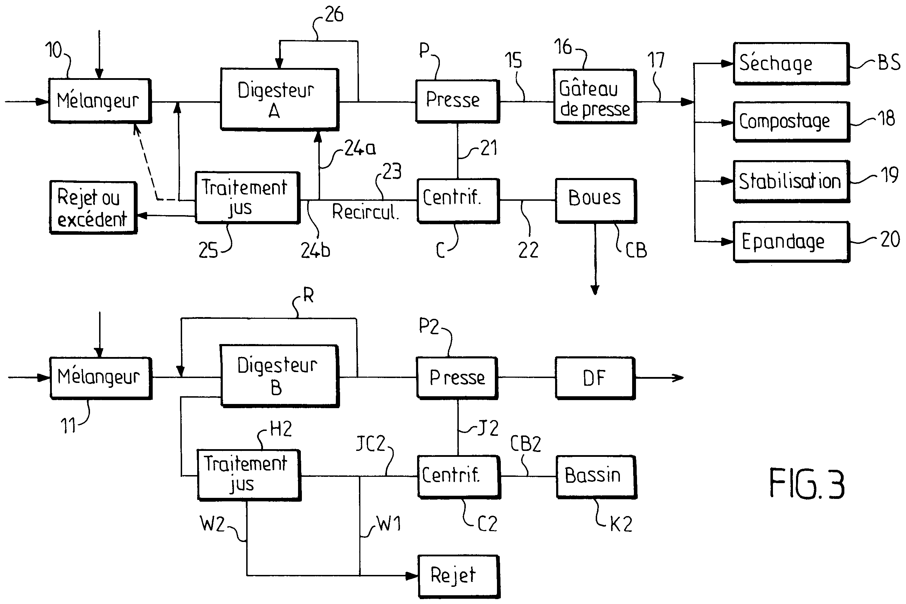

Les produits mélangés sortant du mélangeur 10 sont introduits dans un ou plusieurs digesteurs A (Fig. 3) pour y subir un traitement de méthanisation. Le digestat sortant du digesteur A est envoyé dans la presse P dont on extrait, par une sortie 15, un gâteau de presse 16 constitué de matières solides hétérogènes humides. Ces matières 16 sont dirigées depuis une sortie 17 soit vers un tunnel de bioséchage BS, soit vers un compostage 18, ou une stabilisation 19, ou un épandage 20.

Le jus 21, ou pressat, sortant de la presse P est dirigé vers une centrifugeuse C. La centrifugeuse C délivre, sur une sortie 22, des boues de centrifugation CB, floculées ou non, ainsi qu'un jus 23 dont une partie 24a est recirculée dans le digesteur A, tandis qu'une autre partie 24b est recirculée à l'entrée du digesteur A, dans le mélangeur 10, comme illustré en tirets sur Fig.3, ou en aval, après avoir éventuellement traversé une unité 25 de traitement des jus, afin d'extraire principalement l'ammoniaque et d'éliminer la phase liquide excédentaire. Une fraction 26 des digestats sortant du digesteur A peut également être recirculée.

L'ensemble des trajets suivis par les produits introduits dans le digesteur A et traités en sortie de ce digesteur constitue une première branche de la ligne de traitement des matières dont la granulométrie est comprise entre 0 et 60 mm, sans avoir subi de fermentation, et des matières dont la granulométrie est comprise entre 10 et 20 mm après passage dans le fermenteur 5 de Fig.2. Une autre branche de la ligne de traitement correspond au mélangeur 11 qui peut être remplacé par une unité tampon servant à un stockage intermédiaire, ou à une unité d'hydrolyse en voie humide.

Les produits sortant du mélangeur 11 (Fig.3) ou de l'unité évoquée ci-dessus, sont dirigés vers un digesteur ou un ensemble de digesteurs B pour y subir une méthanisation. Le digestat sortant du digesteur B est généralement dirigé vers une presse P2, une fraction R de ce digestat étant recirculée en tête du digesteur B. Le jus J2 de pressage est envoyé dans une centrifugeuse C2. Le jus JC2 sortant de cette centrifugeuse C2 peut être recirculé à l'entrée du digesteur B après passage dans une unité H2 de traitement des jus. Des fractions W1, W2 du jus prélevées en amont et en aval de l'unité H2 peuvent être envoyées au rejet. Les boues de centrifugation produites par la centrifugeuse C2 sont envoyées dans un bassin K2.

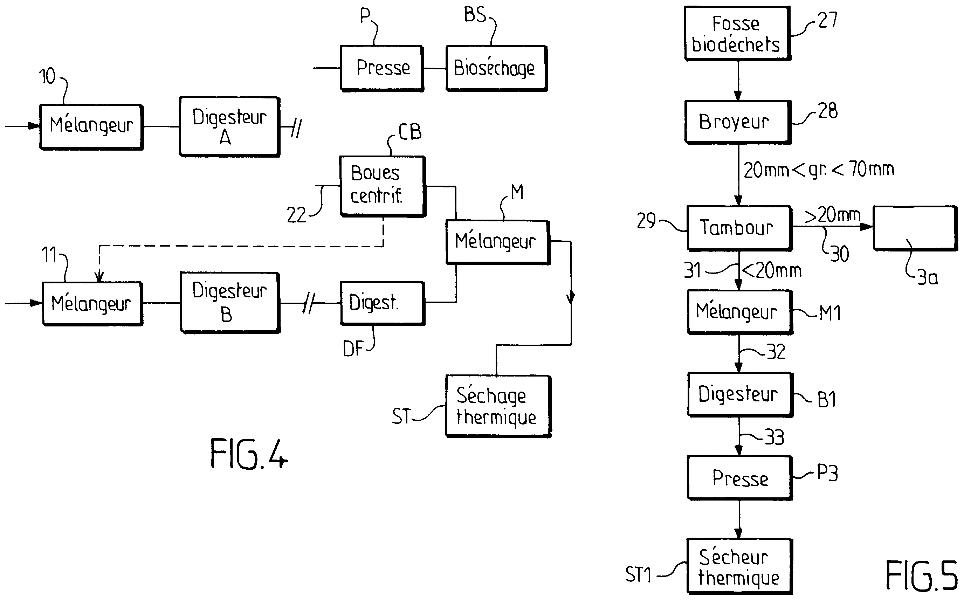

Les matières solides sortant de la presse P2 forment un digestat fin DF. Comme illustré sur Fig. 4, le digestat DF provenant de la deuxième branche de la ligne de traitement comportant le digesteur B est mélangé, dans un mélangeur M, avec les boues de centrifugation CB, ou le pressat, de l'autre branche de la ligne de traitement.

Le mélange issu du mélangeur M est introduit dans un sécheur thermique ST basse température intensif. La basse température désigne une température inférieure à 95°C, de préférence inférieure à 85°C et supérieure à 60°C. Le séchage est réalisé avec de l'air chaud circulant partiellement en circuit fermé.

Les boues de centrifugation recueillies dans le bassin K2 (Fig.3) peuvent être également mélangées dans le mélangeur M au digestat DF et aux boues de centrifugation CB de la première branche de ligne de traitement. Le mélange ainsi réalisé permet d'obtenir un produit acceptable par un sécheur thermique basse température intensif ST.

En variante, les boues de centrifugation recueillies dans le bassin K2 peuvent être renvoyées en amont du digesteur B.

Il est apparu particulièrement intéressant de mélanger et de sécher ensemble le digestat DF et les boues de centrifugation CB pour fixer, au niveau où on le souhaite, la siccité finale des boues séchées et pour minimiser ainsi la consommation énergétique. La présence d'éléments fibreux dans le digestat permet de structurer les boues séchées et d'éviter le passage par une phase collante en cours de séchage qui obligerait à monter jusqu'à 85 % minimum la siccité finale, ce qui serait le cas pour des boues de centrifugation seules. Il s'agit d'un résultat surprenant et inattendu procuré par l'invention.

Le séchage par sécheur thermique intensif basse température ST, du mélange réalisé avec un digestat brut issu de produits dont la granulométrie est de 0-10 mm et de boues de centrifugation, s'est révélé possible alors que, selon les prescriptions usuelles, un sécheur thermique basse température ne doit pas être utilisé avec un digestat sensiblement hétérogène. La granulométrie du mélange obtenu entre le digestat DF et les boues de centrifugation CB et éventuellement CB2 convient à un procédé d'extrusion des produits après séchage.

Le ou les sécheur(s) thermique(s) ST comporte(nt) généralement des batteries de condensation pour récupérer de l'énergie, sous forme d'eau chaude, notamment à 60°C, utilisée pour chauffer l'air des tunnels de maturation ou de bioséchage.

Selon l'invention, le digestat fin sortant du digesteur B ou B1 peut être introduit directement dans le mélangeur M, ou le sécheur thermique, sans être déshydraté au préalable, notamment par pressage et/ou centrifugation. Le digestat brut 0-10 mm non déshydraté, sortant du digesteur B ou B1, et n'ayant pas subi de pressage et donc pas d'ajout de structurants sous forme de déchets verts, présente généralement une siccité de 25 à 30 % avant d'entrer dans le sécheur, et une granulométrie inférieure à 10 mm. Il s'agit d'un produit relativement homogène et fin.

Les boues de centrifugation non floculées présentent une siccité de 35 à 40 %, pour une granulométrie inférieure à 5 mm. Il s'agit également d'un produit homogène et fin. Les boues de centrifugation floculées présentent une siccité d'environ 30 % pour une granulométrie inférieure à 5 mm. Il s'agit également d'un produit homogène et fin.

La préparation du produit en amont du sécheur thermique intensif formé par le digestat fin, et généralement mélangé aux boues de centrifugation, conduit à un aspect et à une granulométrie compatibles avec un procédé d'extrusion pratiqué avant d'introduire les matières à sécher dans le sécheur thermique. Pour du digestat hétérogène, la granulométrie n'est pas compatible avec une telle extrusion.

Le digestat, déshydraté ou non, contient beaucoup d'éléments fibreux : il n'y a pas de changement d'état de la phase liquide à la phase solide. Il est possible d'arrêter le séchage, assuré par le sécheur thermique intensif, à la siccité voulue, de 60 à 95 %. Le produit résultant ne collera pas et sera facilement manipulable.

Fig. 5 est un schéma bloc d'une ligne de traitement selon l'invention dédiée uniquement à des biodéchets recueillis notamment dans des poubelles dites organiques. Cette ligne de traitement comporte une zone de réception 27 constituée par une dalle ou une fosse. Un moyen de transfert non représenté prélève des matières dans la zone de réception pour les introduire dans un broyeur 28 donnant en sortie des matières dont la granulométrie est comprise entre 20 et 70 mm, avantageusement de l'ordre de 50 mm. Les matières sortant du broyeur 28 sont dirigées vers un tambour séparateur 29 à mailles de 20 mm. Le refus 30, d'une granulométrie supérieure à 20 mm, est dirigé vers une unité de valorisation énergétique 3a. Les passants 31, d'une granulométrie inférieure à 20 mm, sont dirigés vers un mélangeur M1 qui permet de réguler l'alimentation du digesteur. La sortie 32 du mélangeur est reliée à un digesteur B1 pour la méthanisation des matières provenant des biodéchets.

Le digesteur B1, comme les digesteurs A et B, comporte une sortie de biogaz non représentée sur le schéma. Le digestat qui sort par une conduite 33 est envoyé à un sécheur thermique intensif ST1, après un passage éventuel dans une presse P3. Avant d'entrer dans le sécheur thermique ST1, la matière est avantageusement soumise à une extrusion, dans un extrudeur non représenté, pour être conditionnée sous forme de cordons d'une longueur de quelques centimètres qui sont introduits dans le sécheur thermique ST1. Ce conditionnement de la matière favorise le séchage.

Le tri des matières effectué selon l'invention en amont des digesteurs permet de produire avec le digesteur B ou B1, traitant les matières de faible granulométrie, un digestat fin de rhéologie adaptée à un sécheur thermique intensif.

Ainsi, à partir de matières hétérogènes contenant des inertes et des métaux, ne pouvant convenir a priori pour un sécheur thermique intensif basse température, l'invention permet de traiter près de la moitié des déchets par un sécheur thermique intensif basse température remplaçant un ou plusieurs tunnels de bioséchage dont l'emprise foncière est beaucoup plus importante.

Les digestats 0-8 mm jusqu'à 0-10 mm, et les refus de centrifugation mélangés constituent des produits triés et fins qui peuvent être soumis à un séchage thermique intensif basse température, alors que les produits hétérogènes correspondant aux granulométries de 0-60 mm et 10-20 mm sont dirigés vers des tunnels de bioséchage.

Or, un seul sécheur thermique intensif basse température peut avoir la capacité de séchage de quatre ou cinq tunnels de bioséchage. La solution de l'invention permet de diminuer de plus de la moitié le nombre de tunnels de bioséchage par la mise en place des sécheurs thermiques intensifs basse température pour les digestats les plus fins associés à l'ensemble des reflux de centrifugation. Le débit d'air de séchage est canalisé et dirigé vers la désodorisation. L'ambiance de travail est considérablement améliorée par rapport à un compostage et à un tunnel de bioséchage.

La quantité d'effluents récupérée dans une ligne de traitement avec sécheur thermique est la même, à siccité finale identique, que dans une ligne de traitement classique avec tunnel de bioséchage. Toutefois, le fait de ne pas presser induit une production des jus centrifugés plus faible et il peut être envisagé de réduire ou même de supprimer le traitement biologique des jus de presse d'où un gain de place supplémentaire.

Les condensats de séchage du sécheur thermique sont peu chargés en MES (matières en suspension) et relativement peu chargés en azote, et sont plus faciles à traiter que les jus de presse. Un traitement physique, notamment par évapoconcentration, peut être envisagé pour résoudre à moindre coût et dans un espace réduit les effluents excédentaires.

Les surfaces économisées avec une ligne de traitement à sécheur thermique intensif sont constituées :
- des surfaces qui auraient été occupées par les tunnels de bioséchage pour le digestat fin 0-10 mm et les boues de centrifugation ;
- de la surface du hall de déshydratation du digestat 0-10 mm ;
- de la surface du hall d'affinage ;
- de la réduction de taille de l'unité de traitement d'eau ;
- de la réduction de la taille de l'unité de désodorisation.

## Revendications

1. Ligne de traitement de déchets constitués d'ordures ménagères résiduelles et/ou de biodéchets, en vue de séparer la matière organique et les matières à haut PCI comprenant :
- des moyens de stockage (1) des déchets,
- des moyens de séparation (2) de matières valorisables,
- des moyens de tri (4, 8, 9, 8a) entre des produits de granulométries différentes,
- des moyens pour diriger les produits de plus forte granulométrie vers au moins une unité de traitement (3), notamment de valorisation énergétique,
- et au moins un digesteur (B, B1) pour la méthanisation de produits à plus forte teneur organique, de granulométrie plus faible,
**caractérisée en ce que** :
- les moyens de tri sont prévus pour fournir une fraction de produits de granulométrie inférieure à 20 mm,
- au moins un digesteur (B, B1) est affecté à la méthanisation de cette fraction de granulométrie inférieure à 20 mm,
- et la ligne de traitement comprend au moins un sécheur thermique basse température (ST, ST1), généralement inférieure à 95°C et supérieure à 60°C, qui est alimenté par le digestat issu de la fraction de granulométrie inférieure à 20 mm.

2. Ligne de traitement selon la revendication 1, **caractérisée en ce que** les moyens de tri sont prévus pour fournir une fraction de produits de granulométrie 0-10 mm, au moins un digesteur (B) est affecté à la méthanisation de cette fraction de granulométrie 0-10 mm, et le sécheur thermique basse température (ST) est alimenté par le digestat de ce digesteur (B).

3. Ligne de traitement selon la revendication 1 ou 2, **caractérisée en ce qu'**elle comporte :
▪ une première branche pour les matières de granulométrie supérieure, comprenant :
- au moins un premier digesteur (A) affecté à la méthanisation desdites matières de granulométrie supérieure,
- au moins une centrifugeuse (C) et/ou une presse (P) à laquelle est soumis le digestat du premier digesteur,
- au moins un tunnel de bioséchage (BS) pour les matières issues de la centrifugeuse ou de la presse,
▪ une deuxième branche pour les matières de granulométrie inférieure, comprenant :
- au moins un deuxième digesteur (B) affecté à la méthanisation des matières de granulométrie inférieure,
- au moins un mélangeur (M) entre le digestat du deuxième digesteur (B) et les boues (CB) de la centrifugeuse, ou le pressat, de la première branche,
- et au moins un sécheur thermique intensif (ST) auquel est soumis le mélange issu du mélangeur.

4. Ligne de traitement selon l'une quelconque des revendications précédentes, **caractérisée en ce qu'**elle comporte :
- des moyens de tri (4,8,9,8a) assurant une production de fractions 0-60 mm, 10-20 mm, et 0-10 mm.
- au moins un premier digesteur (A) pour les fractions 0-60 mm et 10-20 mm qui sont méthanisées avec du structurant, notamment fibres végétales, puis soumises à une presse (P) et/ou une centrifugeuse (C) pour la production de gâteaux de presse et/ou de boues de centrifugation (CB),
- au moins un deuxième digesteur (B) pour la fraction 0-10 mm qui est méthanisée sans structurant et n'est pas déshydratée en sortie,
- un mélangeur (M) pour mélanger le digestat issu du deuxième digesteur (B) avec les boues de centrifugation (CB) du digestat provenant du premier digesteur (A)
- au moins un sécheur thermique intensif basse température (ST) dans lequel est séché le mélange sortant du mélangeur (M) ou du digesteur (B),
- et au moins un tunnel de maturation/bioséchage pour le gâteau de presse de la fraction 0-60 mm et 10-20 mm.

5. Ligne de traitement selon la revendication 4, **caractérisée en ce qu'**elle comporte :
- un premier tambour séparateur (2) dont les mailles correspondent à une granulométrie comprise entre 100 et 500 mm, notamment égale à 300 mm
- un poste (3) de valorisation énergétique du refus du premier tambour séparateur (2),
- un deuxième tambour séparateur (4) à mailles plus petites que celles du premier tambour (2), notamment à mailles de 60 mm, auquel sont envoyés les passants du premier tambour (2),
- un fermenteur (5) auquel est envoyé le refus du deuxième tambour (4), après être passé par une étape de tri (6) qui extrait les produits métalliques, notamment les produits ferreux,
- un troisième tambour séparateur (7), dont les mailles correspondent à une granulométrie de 20 mm, qui reçoit les produits sortant du fermenteur (5), le refus du troisième tambour (7) étant dirigé vers le poste (3) de valorisation énergétique,
- des moyens (8, 9, 8a) pour séparer les passants, ayant traversé les mailles du troisième tambour (7), en une fraction de granulométrie comprise entre 10 et 20 mm, dirigée vers un mélangeur (10) et une fraction de granulométrie 0-10 mm, dirigée vers le deuxième digesteur (B).

6. Ligne de traitement selon la revendication 5, **caractérisée en ce que** le mélangeur (10), qui reçoit la fraction de granulométrie comprise entre 10 et 20 mm, reçoit également les passants qui ont traversé le deuxième tambour séparateur (4), les produits mélangés sortant du mélangeur (10) étant introduits dans le premier digesteur (A).

7. Ligne de traitement selon la revendication 1 pour biodéchets, **caractérisée en ce qu'**elle comporte :
- un broyeur (28) de biodéchets,
- des moyens de criblage (29) pour fournir une fraction de produits de granulométrie inférieure à 20 mm et une fraction de produits de granulométrie supérieure,
- au moins un digesteur (B1) affecté à la méthanisation des produits de granulométrie inférieure à 20 mm,
- et au moins un sécheur thermique intensif (ST1) auquel est soumis le digestat du digesteur (B1), après traversée éventuelle d'une centrifugeuse ou d'une presse (P3).

8. Ligne de traitement selon l'une quelconque des revendications précédentes, **caractérisée en ce que** le sécheur thermique (ST, ST1) est piloté pour que la siccité finale en sortie du sécheur thermique soit comprise entre 50 et 90 %, en fonction des attentes de l'exploitation.

## Patentansprüche

1. Behandlungsstraße für Abfall, der aus Haushaltsrestmüll und/oder Biomüll besteht, im Hinblick auf eine Trennung der organischen Substanz und der Substanzen mit hohem Heizwert, umfassend:
- Einrichtungen zur Lagerung (1) des Abfalls;
- Einrichtungen zur Abtrennung (2) von Wertstoffen;
- Einrichtungen zur Sortierung (4, 8, 9, 8a) von Produkten unterschiedlicher Korngröße;
- Einrichtungen zur Lenkung der Produkte größerer Korngröße in Richtung wenigstens einer Behandlungseinheit (3), insbesondere zur Energierückgewinnung; und
- wenigstens einen Faulbehälter (B, B1) für die Methanisierung von Produkten mit höherem organischem Anteil mit geringerer Korngröße;
**dadurch gekennzeichnet, dass**
- die Einrichtungen zur Sortierung dazu vorgesehen sind, eine Fraktion von Produkten mit einer Korngröße von unter 20 mm zu liefern;
- wenigstens ein Faulbehälter (B, B1) der Methanisierung dieser Fraktion mit einer Korngröße von unter 20 mm zugeordnet ist;
- und die Behandlungsstraße wenigstens einen thermischen Trockner niederer Temperatur (ST, ST1), die im Allgemeinen unter 95 °C und über 60 °C liegt, umfasst, dem der aus der Fraktion mit einer Korngröße von unter 20 mm hervorgegangene Gärrest zugeführt wird.

2. Behandlungsstraße gemäß Anspruch 1, **dadurch gekennzeichnet, dass** die Einrichtungen zur Sortierung dazu vorgesehen sind, eine Fraktion von Produkten mit einer Korngröße von 0-10 mm zu liefern, wenigstens ein Faulbehälter (B) der Methanisierung dieser Fraktion mit einer Korngröße von 0-10 mm zugeordnet ist und dem thermischen Trockner niederer Temperatur (ST) der Gärrest aus diesem Faulbehälter (B) zugeführt wird.

3. Behandlungsstraße gemäß Anspruch 1 oder 2, **dadurch gekennzeichnet, dass** sie umfasst:
▪ einen ersten Zweig für Substanzen größerer Korngröße, umfassend:
- wenigstens einen ersten Faulbehälter (A), der der Methanisierung dieser Substanzen größerer Korngröße zugeordnet ist;
- wenigstens eine Zentrifuge (C) und/oder eine Presse (P), der der Gärrest des ersten Faulbehälters unterworfen wird;
- wenigstens einen Tunneltrockner zur Biotrocknung (BS) für die aus der Zentrifuge oder Presse hervorgegangenen Substanzen;
▪ einen zweiten Zweig für Substanzen niederer Korngröße, umfassend:
- wenigstens einen zweiten Faulbehälter (B), der der Methanisierung der Substanzen niederer Korngröße zugeordnet ist;
- wenigstens einen Mischer (M) zum Mischen des Gärrests des zweiten Faulbehälters (B) und der Schlämme (CB) der Zentrifuge oder des Pressguts aus dem ersten Zweig; und
- wenigstens einen thermischen Intensivtrockner (ST), dem das aus dem Mischer hervorgegangene Gemisch unterworfen wird.

4. Behandlungsstraße gemäß einem der vorstehenden Ansprüche, **dadurch gekennzeichnet, dass** sie umfasst:
- Einrichtungen zur Sortierung (4, 8, 9, 8a), die eine Produktion von Fraktionen von 0-60 mm, 10-20 mm und 0-10 mm gewährleisten;
- wenigstens einen ersten Faulbehälter (A) für die Fraktionen von 0-60 mm und 10-20 mm, die mit einem Strukturierungsmittel, insbesondere Pflanzenfasern, methanisiert werden, dann einer Presse (P) und/oder einer Zentrifuge (C) unterworfen werden, wobei Presskuchen und/oder Zentrifugationsschlämme (CB) entstehen;
- wenigstens einen zweiten Faulbehälter (B) für die Fraktion von 0-10 mm, die ohne Strukturierungsmittel methanisiert und im Ausgang nicht entwässert wird;
- einen Mischer (M) zum Mischen des aus dem zweiten Faulbehälter (B) hervorgegangenen Gärrests mit den Zentrifugationsschlämmen (CB) des aus dem ersten Faulbehälter (A) hervorgegangenen Gärrests;
- wenigstens einen thermischen Niedertemperatur-Intensivtrockner (ST), in dem das aus dem Mischer (M) oder dem Faulbehälter (B) austretende Gemisch getrocknet wird; und
- wenigstens einen Tunnel zur Reifung/Biotrocknung für den Presskuchen der Fraktion von 0-60 mm und 10-20 mm.

5. Behandlungsstraße gemäß Anspruch 4, **dadurch gekennzeichnet, dass** sie umfasst:
- eine erste Trenntrommel (2), deren Maschen einer Korngröße zwischen 100 und 500 mm, insbesondere gleich 300 mm, entsprechen;
- eine Station (3) zur Energierückgewinnung aus dem Rückstand der ersten Trenntrommel (2);
- eine zweite Trenntrommel (4) mit Maschen, die kleiner sind als die der ersten Trommel (2), insbesondere mit Maschen von 60 mm, der der Durchgang der ersten Trommel (2) zugeführt wird;
- einen Gärbehälter (5), dem der Rückstand der zweiten Trommel (4) zugeführt wird, nachdem er einen Sortierungsschritt (6), der die metallischen Produkte, insbesondere die eisenhaltigen Produkte, herauszieht, durchlaufen hat;
- eine dritte Trenntrommel (7), deren Maschen einer Korngröße von 20 mm entsprechen und die aus dem Gärbehälter (5) austretenden Produkte aufnimmt, wobei der Rückstand der dritten Trommel (7) der Station (3) zur Energierückgewinnung zugeleitet wird;
- Einrichtungen (8, 9, 8a) zur Auftrennung des Durchgangs, der die Maschen der dritten Trommel (7) passiert hat, in eine Fraktion mit einer Korngröße zwischen 10 und 20 mm, die einem Mischer (10) zugeleitet wird, und eine Fraktion mit einer Korngröße von 0-10 mm, die dem zweiten Faulbehälter (B) zugeleitet wird.

6. Behandlungsstraße gemäß Anspruch 5, **dadurch gekennzeichnet, dass** der Mischer (10), der die Fraktion mit einer Korngröße zwischen 10 und 20 mm aufnimmt, auch den Durchgang der zweiten Trenntrommel (4) aufnimmt, wobei die gemischten Produkte, die aus dem Mischer (10) austreten, in den ersten Faulbehälter (A) eingeführt werden.

7. Behandlungsstraße gemäß Anspruch 1 für Biomüll, **dadurch gekennzeichnet, dass** sie umfasst:
- einen Häcksler (28) für Biomüll;
- Siebeinrichtungen (29), die eine Fraktion von Produkten mit einer Korngröße unter 20 mm und eine Fraktion von Produkten mit größerer Korngröße liefern;
- wenigstens einen Faulbehälter (B1), der der Methanisierung der Produkte mit einer Korngröße von unter 20 mm zugeordnet ist; und
- wenigstens einen thermischen Intensivtrockner (ST1), dem der Gärrest des Faulbehälters (B1) unterworfen wird, nachdem es gegebenenfalls eine Zentrifuge oder Presse (P3) durchlaufen hat.

8. Behandlungsstraße gemäß einem der vorstehenden Ansprüche, **dadurch gekennzeichnet, dass** der thermische Trockner (ST, ST1) so gesteuert wird, dass die Endtrockenheit am Ausgang des thermischen Trockners in Abhängigkeit von den Nutzungserwartungen zwischen 50 und 90% liegt.

## Claims

1. A treatment line for treating waste consisting of residual household waste and/or biowaste, for the purpose of separating the organic matter and the matters with a high LHV, comprising:
- means (1) for storing waste,
- means (2) for separating recoverable matters,
- means (4, 8, 9, 8a) for sorting products having different particle sizes,
- means for directing the products of larger particle size to at least one treatment unit (3), in particular for energy recovery,
- and at least one digester (B, B1) for the methanization of products with a higher organic content, having a smaller particle size,
**characterized in that**:
- the sorting means are provided in order to supply a fraction of products having a particle size of less than 20 mm,
- at least one digester (B, B1) is used for the methanization of this fraction having a particle size of less than 20 mm,
- and the treatment line comprises at least one low-temperature, generally below 95°C and above 60°C, thermal dryer (ST, ST1), which is fed with the digestate derived from the fraction having a particle size of less than 20 mm.

2. The treatment line as claimed in claim 1, **characterized in that** the sorting means are provided in order to supply a fraction of products having a particle size of 0-10 mm, at least one digester (B) is used for the methanization of this fraction having a particle size of 0-10 mm, and the low-temperature thermal dryer (ST) is fed with the digestate from this digester (B).

3. The treatment line as claimed in claim 1 or 2, **characterized in that** it comprises:
▪ a first branch for the matters having a larger particle size, comprising:
- at least one first digester (A) used for the methanization of said matters having a larger particle size,
- at least one centrifuge (C) and/or one press (P) to which the digestate from the first digester is subjected;
- at least one biodrying tunnel (BS) for the matters from the centrifuge or from the press,
▪ a second branch for the matters having a smaller particle size, comprising:
- at least one second digester (B) used for the methanization of the matters having a smaller particle size,
- at least one mixer (M) between the digestate of the second digester (B) and the sludge (CB) from the centrifuge, or the pressate, of the first branch,
- and at least one intensive thermal dryer (ST) to which the mixture from the mixer is subjected.

4. The treatment line as claimed in any one of the preceding claims, **characterized in that** it comprises:
- sorting means (4, 8, 9, 8a) providing a production of 0-60 mm, 10-20 mm and 0-10 mm fractions,
- at least one first digester (A) for the 0-60 mm and 10-20 mm fractions which are methanized with structuring agent, in particular vegetable fibers, then subjected to a press (P) and/or a centrifuge (C) for the production of presscakes and/or of centrifugation sludge (CB),
- at least one second digester (B) for the 0-10 mm fraction which is methanized without structuring agent and is not dehydrated at the outlet,
- a mixer (M) for mixing the digestate from the second digester (B) with the sludge from centrifugation (CB) of the digestate originating from the first digester (A),
- at least one low-temperature intensive thermal dryer (ST) in which the mixture exiting the mixer (M) or the digester (B) is dried,
- and at least one maturing/biodrying tunnel for the presscake of the 0-60 mm and 10-20 mm fraction.

5. The treatment line as claimed in claim 4, **characterized in that** it comprises:
- a first separating drum (2), the meshes of which correspond to a particle size of between 100 and 500 mm, in particular equal to 300 mm,
- an energy recovery station (3) for recovering energy from the oversize of the first separating drum (2),
- a second separating drum (4) with meshes that are smaller than those of the first drum (2), in particular with meshes of 60 mm, to which the undersize of the first drum (2) is sent,
- a fermentor (5) to which the oversize of the second drum (4) is sent, after having gone through a sorting step (6) which extracts the metallic products, in particular the ferrous products,
- a third separating drum (7), the meshes of which correspond to a particle size of 20 mm, which receives the products exiting the fermentor (5), the oversize of the third drum (7) being directed to the energy recovery station (3),
- means (8, 9, 8a) for separating the undersize, having passed through the meshes of the third drum (7), into a fraction having a particle size of between 10 and 20 mm, directed to a mixer (10), and a fraction having a particle size of 0-10 mm, directed to the second digester (B).

6. The treatment line as claimed in claim 5, **characterized in that** the mixer (10) which receives the fraction having a particle size of between 10 and 20 mm, also receives the undersize which has passed through the second separating drum (4), the mixed products exiting the mixer (10) being introduced into the first digester (A).

7. The treatment line as claimed in claim 1, for biowaste, **characterized in that** it comprises:
- a biowaste grinder (28),
- screening means (29) for supplying a fraction of products having a particle size of less than 20 mm and a fraction of products having a larger particle size,
- at least one digester (B1) used for the methanization of the products having a particle size of less than 20 mm,
- and at least one intensive thermal dryer (ST1) to which the digestate from the digester (B1) is subjected after optionally being passed through a centrifuge or a press (P3).

8. The treatment line as claimed in any one of the preceding claims, **characterized in that** the thermal dryer (ST, ST1) is controlled such that the final dryness at the outlet of the thermal dryer is between 50% and 90%, according to the expectations of the operation.
